# EUROPÄISCHE PATENTSCHRIFT

(11) **EP 1 752 107 B1**
(45) Veröffentlichungstag und Bekanntmachung des Hinweises auf die Patenterteilung: **23.06.2010**
(21) Anmeldenummer: 06016498.5
(22) Anmeldetag: 08.08.2006
(51) Int. Cl.: A61B 18/14, A61B 18/18

(54) **Medizinisches Instrument**
Medical instrument
Instrument médical

(30) Priorität: 12.08.2005 DE 102005039106
(43) Veröffentlichungstag der Anmeldung: 14.02.2007
(73) Patentinhaber: Karl Storz GmbH & Co. KG, 78532 Tuttlingen (DE)
(72) Erfinder: Hahn, Martin, 88605 Boll (DE)
(74) Vertreter: Heuckeroth, Volker

(56) Entgegenhaltungen:
- EP-A- 0 084 419
- DE-A1- 4 425 015
- DE-C1- 19 630 666
- US-A- 5 857 962
- US-A- 5 993 445
- US-B1- 6 358 200

## Beschreibung

Die Erfindung betrifft ein medizinisches Instrument, insbesondere Resektoskop, mit einem ersten Arbeitselement zur Behandlung von Körpergewebe, das über einen langerstreckten Arbeitselementträger mit einer Handhabe einem proximalen Ende zum Bewegen des Arbeitselements verbindbar ist, wobei die Handhabe einen beweglichen Schlitten aufweist, der eine erste Aufnahme zum lösbaren Festlegen des Arbeitselementträgers an dem Schlitten aufweist, und mit einer Aufnahme für ein Endoskop, dessen Endoskopschaft sich im montierten Zustand im Wesentlichen parallel zum ersten Arbeitselementträger erstreckt; wobei der Schlitten zumindest eine von der ersten Aufnahme beabstandete zweite Aufnahme für den ersten Arbeitselementträger oder zumindest einen zweiten, am distalen Ende zumindest ein zweites Arbeitselement tragenden Arbeitselementträger aufweist, wobei die erste Aufnahme des Schlittens mit einer elektrischen Kontaktierung versehen ist und die zweite Aufnahme des Schlittens gegen die erste Aufnahme elektrisch isoliert ist.

Ein derartiges Instrument ist aus dem Dokument US-A-5 993 445 bekannt.

Ein medizinisches Instrument der eingangs genannten Art wird im Rahmen der minimal-inversiven Chirurgie insbesondere auf dem Gebiet der Urologie verwendet. In der Urologie wird ein solches Instrument dazu benutzt, Gewebe, bspw. innerhalb der Harnröhre, zu präparieren, insbesondere zu schneiden. Ein solches Instrument wird auch als Resektoskop bezeichnet, wobei jedoch die vorliegende Erfindung nicht auf ein Resektoskop beschränkt ist. Das Instrument ist üblicherweise mit einem Endoskop kombiniert.

Das aus dem oben genannten Dokument bekannte Instrument ist ein Resektoskop, das ein erstes Arbeitselement in Form einer Schneidelektrode und ein zweites Arbeitselement in Form einer Koagulationselektrode aufweist. Die Schneidelektrode und die Koagulationselektrode sind mit jeweils einem langerstreckten Arbeitselementträger mit der Handhabe am proximalen Ende des Instruments verbindbar, wobei die Handhabe einen beweglichen Schlitten aufweist, in der der Arbeistelementträger mit der Schneidelektrode und der Arbeitselementträger mit der Koagulationselektrode gleichzeitig verbunden werden.

Aus dem Dokument DE-A-103 45 111 ist ein Instrument bekannt, das als Arbeitselement eine Elektrode aufweist, die an einem Elektrodenträger als Arbeitselementträger am distalen Ende angeordnet ist. Die Elektrode selbst weist bspw. die Form einer Resektionsschlinge auf. Mit einer solchen Elektrode, bspw. in Form einer Resektionsschlinge, wird Gewebe unter der Wirkung von Hochfrequenzstrom mit dem die Elektrode beaufschlagt wird, ziehend geschnitten. Eine solche Elektrode kann jedoch unter der Wirkung von Hochfrequenzstrom nicht nur schneidend, sondern bei entsprechender Formgebung auch koagulierend oder vaporisierend wirken. In dem genannten Dokument wird beschrieben, dass anstelle von mit Hochfrequenzstrom beaufschlagbaren Arbeitselementen auch stromlos arbeitende Instrumente wie bspw. Messer, Küretten usw. bei dem Instrument verwendet werden können.

Bei dem bekannten Instrument ist am proximalen Ende eine Handhabe vorgesehen, die einen Schlitten aufweist, der eine Aufnahme für das proximale Ende des Arbeitselementträgers aufweist. Im Fall, dass der Arbeitselementträger am distalen Ende eine Elektrode trägt, wird das proximale Ende des Arbeitselementträgers am Schlitten außerdem elektrisch kontaktiert, d.h. im festgelegten Zustand mit dem Anschluss für ein Stromkabel leitend verbunden.

Bei derartigen Instrumenten ist es üblich, dass eine Mehrzahl unterschiedlicher Arbeitselemente mit jeweiligem Arbeitselementträger bereitgestellt werden, die vom Arzt nach Bedarf gegeneinander ausgetauscht und in der Aufnahme des Schlittens festlegbar sind. Je nach Vorliebe des das Instrument bedienenden Arztes wird es teilweise vorgezogen, Gewebe mit einer Elektrode, also unter der Wirkung von Hochfrequenzstrom zu schneiden, oder aber stromlos auf rein mechanische Weise, bspw. mit einem Messer. Soll bspw. ein stromlos arbeitendes Messer anstelle der Elektrode zum Schneiden von Gewebe verwendet werden, wird bei dem bekannten Instrument die Elektrode samt ihrem Arbeitselementträger (Elektrodenträger) von dem Schlitten gelöst und entnommen, und das Messer mit seinem Arbeitselementträger an dem Schlitten in der selben Aufnahme festgelegt. Dies hat zur Folge, dass alle vorhandenen verschiedenen Arbeitselemente und die mit diesen verbundenen Arbeitselementträger stets an der selben Stelle des Schlittens festgelegt werden. Damit ist die Lage des Arbeitselementes am distalen Ende des Arbeitselementträgers, die durch die Formgebung des Arbeitselementes und des Arbeitselementträgers bestimmt ist, stets fest vorgegeben, insbesondere die Lage des Arbeitselements relativ zum distalen Sichtfenster des Endoskops und damit zum Blickfeld des Endoskops. Wenn es also gewünscht ist, das selbe Arbeitselement, bspw. ein Messer oder eine Resektionsschlinge, in verschiedenen räumlichen Lagen, insbesondere zum Blickfeld des Endoskops, verwenden zu können, ist es bei dem bekannten Instrument erforderlich, eine selbe Art von Arbeitselement, bspw. eine Resektionsschlinge, in einer Mehrzahl unterschiedlicher geometrischer Ausgestaltungen des Arbeitselements und des Arbeitselementträgers vorzuhalten. Dies erhöht allerdings den Kostenaufwand eines solchen Instruments.

Aus dem Dokument EP-A-0 084 419 ist ein medizinisches Instrument in Form eines Resektoskops bekannt, das eine Elektrode am distalen Ende aufweist, die über einen Arbeitselementträger mit einem axial beweglichen Schlitten verbunden ist. Der Schlitten weist ein flexibles Element auf, in dem ein Schlitz vorhanden ist, der an einem Ende eine kreisförmige Aufnahme für das proximale Ende des Arbeitselementträgers der Elektrode, und am anderen Ende eine weitere kreisförmige Öffnung aufweist, die dazu vorgesehen ist, die Komprimierbarkeit des flexiblen Elements zum Festklemmen des proximalen Endes des Arbeitselementträgers der Elektrode zu erhöhen.

US-A-5 857 962 offenbart ebenfalls ein Resektoskop, das eine Elektrode aufweist, die über einen Arbeitselementträger mit einem axial beweglichen Schlitten verbunden ist. Wie bei dem aus dem vorstehend genannten Dokument bekannten Instrument weist der Schlitten nur eine Aufnahme zur Aufnahme des Arbeitselementträgers auf.

Das Dokument DE-A-42 42 143 offenbart ein hochfrequenzchirurgisches Multifunktions-Handinstrument zum bipolaren Koagulieren und Schneiden sowie zum Spülen des Operationsgebietes und zum Absaugen von Flüssigkeit und Gewebeteilchen bei endoskopischen Operationen, das an einen Hochfrequenzgenerator sowie Spül- und Saugmittel anschließbar ist. Bei diesem bekannten Instrument sind gleichzeitig eine Hochfrequenz-Koagulationssonde mit zwei Koagulationselektroden sowie eine Hochfrequenz-Schneidsonde mit einer Schneidnadel oder Schneidschlinge und einer dagegen isolierten Gegenelektrode am Instrument festlegbar. Den beiden Hochfrequenzsonden ist jeweils ein Schieber an der Handhabe des Instruments zugeordnet, so dass die beiden Sonden wahlweise und einzeln axial bewegt werden können, um einen Behandlungsvorgang vorzunehmen. Wie die beiden Sonden an den Schiebern festgelegt sind, ist nicht offenbart.

Der vorliegenden Erfindung liegt die Aufgabe zu Grunde, ein medizinisches Instrument der eingangs genannten Art hinsichtlich seiner Funktionalität möglichst ohne erhöhten Kostenaufwand zu verbessern.

Erfindungsgemäß wird diese Aufgabe hinsichtlich des eingangs genannten medizinischen Instruments dadurch gelöst, dass die zweite Aufnahme keine elektrische Kontaktierung aufweist.

Bei dem erfindungsgemäßen Instrument ist es somit möglich, ein und denselben Arbeitselementträger mit ein und demselben Arbeitselement wahlweise in der ersten Aufnahme oder der zumindest zweiten oder jeder weiteren vorgesehenen Aufnahme festzulegen, wodurch der Arzt die für ihn günstigste räumliche Lage des Arbeitselements am distalen Ende, insbesondere zum Blickfeld des Endoskops wählen kann. Es ist aber auch möglich, die erste Aufnahme für eine erste Art von Arbeitselementen zu benutzen, bspw. für mit Hochfrequenzstrom beaufschlagbare Arbeitselemente, und die zumindest zweite und ggf. weiteren vorgesehenen Aufnahmen für eine andere Art von Arbeitselementen, insbesondere stromlos arbeitende Arbeitselemente, bspw. Messer, Küretten und dergleichen. Durch das vorsehen zumindest einer zweiten Aufnahme am Schlitten wird die Funktionalität des erfindungsgemäßen Instruments ohne nennenswerten höheren Kostenaufwand realisiert. Dadurch, dass die zumindest zweite Aufnahme ebenfalls am Schlitten vorgesehen ist, der über Betätigung der Handhabe zur Bewegung des Arbeitselements am distalen Ende beweglich ist, wird der Betätigungsmechanismus, der bereits für den Schlitten vorhanden ist, vorteilhafterweise auch für die zumindest zweite Aufnahme genutzt, wodurch kein zusätzlicher Konstruktionsaufwand zur Realisierung einer Betätigungseinrichtung für die zumindest zweite Aufnahme entsteht.

Die erste Aufnahme des Schlittens ist mit einer elektrischen Kontaktierung versehen, und die zumindest zweite Aufnahme des Schlittens ist gegen die erste Aufnahme elektrisch isoliert und weist erfindungsgemäß keine elektrische Kontaktierung auf.

Der Vorteil hierbei ist, dass zumindest eine Aufnahme am Schlitten vorgesehen ist, die zur Festlegung eines stromlos arbeitenden Arbeitselements, bspw. eines Messers, vorgesehen ist, die elektrisch isoliert ist. Bei dem aus DE-A-103 45 111 bekannten Instrument ist demgegenüber sowohl für strombeaufschlagte als auch stromlos arbeitende Arbeitselemente ein und dieselbe Aufnahme vorgesehen, wodurch die Gefahr besteht, dass auch die stromlos arbeitenden Arbeitselemente versehentlich mit Strom beaufschlagt werden, was eine Gefährdung des das Instrument bedienenden Arztes oder des Patienten darstellen kann. Diese Gefährdung wird bei dem erfindungsgemäßen Instrument vorteilhafterweise durch das Vorsehen zumindest einer elektrisch isolierten Aufnahme ohne elektrische Kontaktierung vermieden.

In einer konstruktiv vorteilhaft einfachen Weise ist die zumindest zweite Aufnahme des Schlittens gegen die erste Aufnahme des Schlittens mittels eines elektrisch isolierenden Kunststoffs isoliert.

Eine konstruktiv besonders kostengünstige Ausgestaltung des Schlittens mit den zumindest zwei Aufnahmen ist in einer weiteren bevorzugten Ausgestaltung dadurch realisiert, dass der Schlitten insgesamt als Kunststoffkörper ausgebildet ist, in dem in der ersten Aufnahme ein oder mehrere elektrische Kontakte vorhanden sind.

Durch die Fertigung des Schlittens insgesamt als Kunststoffkörper mit bspw. eingebetteten elektrischen Kontakten in der ersten Aufnahme wird bei konstruktiv vorteilhaft einfacher Ausgestaltung eine zuverlässige und durchschlagsichere elektrische Isolierung zwischen den zumindest zwei Aufnahmen gewährleistet.

Während es im Rahmen der vorliegenden Erfindung möglich ist, die erste und die zumindest zweite Aufnahme an dem Schlitten im Abstand zueinander an beliebigen Positionen vorzusehen, ist es in einer weiteren bevorzugten Ausgestaltung vorgesehen, dass die erste und zumindest zweite Aufnahme an dem Schlitten so positioniert sind, dass sich, wenn das Instrument bestimmungsgemäß in der Hand gehalten wird, die erste Aufnahme oberhalb eines Aufnahmerohrs für den Endoskopschaft und die zumindest zweite Aufnahme unterhalb des Aufnahmerohrs für den Endoskopschaft befindet.

"Oberhalb des Aufnahmerohrs für das Endoskop" und "unterhalb des Aufnahmerohrs für das Endoskop" bedeutet vorliegend nicht zwangsläufig, dass die zumindest zwei Aufnahmen für den ersten und/oder zweiten Arbeitselementträger auf axial gleicher oder etwa gleicher Höhe angeordnet sind, sondern in axialer Richtung können die Aufnahmen für den oder die Arbeitselementträger axial bezüglich der Aufnahme für das Endoskop versetzt sein.

Da die insbesondere bei Resektoskopen verwendeten Arbeitselemente, seien es strombeaufschlagbare oder stromlos arbeitende Arbeitselemente, so ausgebildet sind, dass mit ihnen ein ziehender Schnitt ausgeführt werden kann, das Arbeitselement also im Wesentlichen senkrecht zur Zugrichtung und damit zur Richtung des oder der Arbeitselementträger angeordnet sind, hat die vorstehend genannte Maßnahme den Vorteil, dass der oder die Arbeitselementträger bei dieser Positionierung vollständig außerhalb des Blickfelds des Endoskops angeordnet werden können, und im Blickfeld des Endoskops lediglich das distale Arbeitselement zu sehen ist. Mit anderen Worten wird es bei dieser Ausgestaltung ermöglicht, dass der oder die Arbeitselementträger die Sicht durch das Endoskop auf das Behandlungsareal nicht stören.

In näherer Ausgestaltung der zuvor genannten Maßnahme ist es vorzugsweise vorgesehen, dass der erste Arbeitselementträger im am Schlitten festgelegten Zustand, und wenn das Instrument bestimmungsgemäß in der Hand gehalten wird, oberhalb des Endoskopschafts verläuft, und dass das erste Arbeitselement etwa senkrecht zur Längsrichtung des ersten Arbeitselementträgers vom Arbeitselementträger nach unten absteht.

Diese Orientierung des Arbeitselements und Arbeitselementträgers ist vorzugsweise für ein strombeaufschlagbares Instrument vorgesehen, bei dem das Arbeitselement demnach als Elektrode, insbesondere in Form einer Schlinge ausgebildet ist.

Für stromlos arbeitende Arbeitselemente, wie bspw. ein Messer, ist es demgegenüber bevorzugt, wenn der zumindest zweite Arbeitselementträger in am Schlitten festgelegten Zustand, und wenn das Instrument bestimmungsgemäß in der Hand gehalten wird, unterhalb des Endoskopschafts verläuft, wobei dann das zumindest eine zweite Arbeitselement etwa senkrecht zur Längsrichtung des zweiten Arbeitselementträgers vom Arbeitselementträger nach oben absteht.

In beiden Fällen ist im Blickfeld des Endoskops somit im Wesentlichen lediglich das Arbeitselement selbst, d.h. einerseits die Resektionsschlinge bzw. andererseits das Messer, zu sehen, ohne dass die zugeordneten Arbeitselementträger die Sicht auf das Behandlungsareal beeinträchtigen.

In einer weiteren bevorzugten Ausgestaltung weist/weisen die erste Aufnahme und/oder die zumindest zweite Aufnahme des Schlittens einen Rastmechanismus zum Festlegen des ersten oder zumindest zweiten Arbeitselementträgers auf.

Diese an sich bekannte Maßnahme erweist sich auch dann als vorteilhaft, wenn der Schlitten mehr als eine Aufnahme zum Festlegen eines Arbeitselementträgers aufweist.

In diesem Zusammenhang ist bevorzugt, wenn die erste Aufnahme und die zumindest zweite Aufnahme jeweils einen Rastmechanismus aufweisen, denen zusammen ein gemeinsames Bedienelement zum Lösen des ersten bzw. zumindest zweiten Arbeitselementträgers zugeordnet ist.

In der Praxis wird das erfindungsgemäße Instrument zur selben Zeit mit nur einem Arbeitselement verwendet, d.h. es wird zur gleichen Zeit nur ein Arbeitselementträger in einer der Aufnahmen des Schlittens festgelegt. Im Sinne einer konstruktiv einfachen und wenig kostenaufwendigen Bauweise ist es daher vorteilhaft, wenn ein Bedienelement vorgesehen ist, mit dem der Rastmechanismus aller vorgesehenen Aufnahmen gleichzeitig gelöst werden kann, da ohnehin nur in einer Aufnahme ein Arbeitselementträger festgelegt ist.

In einer weiteren bevorzugten Ausgestaltung ist an der Handhabe des Weiteren ein Schaft festlegbar, der im festgelegten Zustand den ersten bzw. zumindest zweiten Arbeitselementträger umgibt, und relativ zu dem der Schlitten axial beweglich ist.

Der Schaft hat den Vorteil, dass das Instrument leichter in natürliche Öffnungen in das Behandlungsgebiet eingeführt werden kann, da nämlich die bei derartigen Instrumenten vorgesehenen langerstreckten Arbeitselementträger sehr dünn und somit relativ flexibel sind, was ein Einführen der Arbeitselementträger mit dem Arbeitselement voran in das Operationsgebiet erschwert.

In einer weiteren bevorzugten Ausgestaltung ist der Schlitten auf einem Führungsrohr axial beweglich gelagert, wobei das Führungsrohr als Aufnahmerohr für den Endoskopschaft dient.

Diese ebenfalls an sich bekannte Maßnahme führt auch bei dem erfindungsgemäßen medizinischen Instrument, das gleichzeitig eine erhöhte Funktionalität auf Grund des Vorhandenseins mehrerer Aufnahmen für einen oder mehrere Arbeitselementträger besitzt, zu einer vorteilhaft einfachen und wenig Teile erfordernden Ausgestaltung.

Weitere Vorteile und Merkmale ergeben sich aus der nachfolgenden Beschreibung der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine Gesamtdarstellung eines medizinischen Instruments in Seitenansicht in einer ersten Betriebsart;
- Fig. 2: das Instrument in Fig. 1 in einem Längsschnitt;
- Fig. 3: eine ausschnittsweise und vergrößerte Darstellung des Instruments in Fig. 1 und 2 im Längsschnitt;
- Fig. 4: eine weitere ausschnittsweise und vergrößerte Darstellung des Instruments in Fig. 1 und 2 im Längsschnitt;
- Fig. 5: eine Gesamtdarstellung des Instruments in Fig. 1 in Seitenansicht in einer zweiten Betriebsart;
- Fig. 6: das Instrument in Fig. 5 im Längsschnitt;
- Fig. 7: eine mit Fig. 3 vergleichbare ausschnittsweise und vergrößerte Darstellung des Instruments in Fig. 5 und 6 im Längsschnitt; und
- Fig. 8: eine mit Fig. 4 vergleichbare ausschnittsweise und vergrößerte Darstellung des Instruments in Fig. 5 und 6 im Längsschnitt.

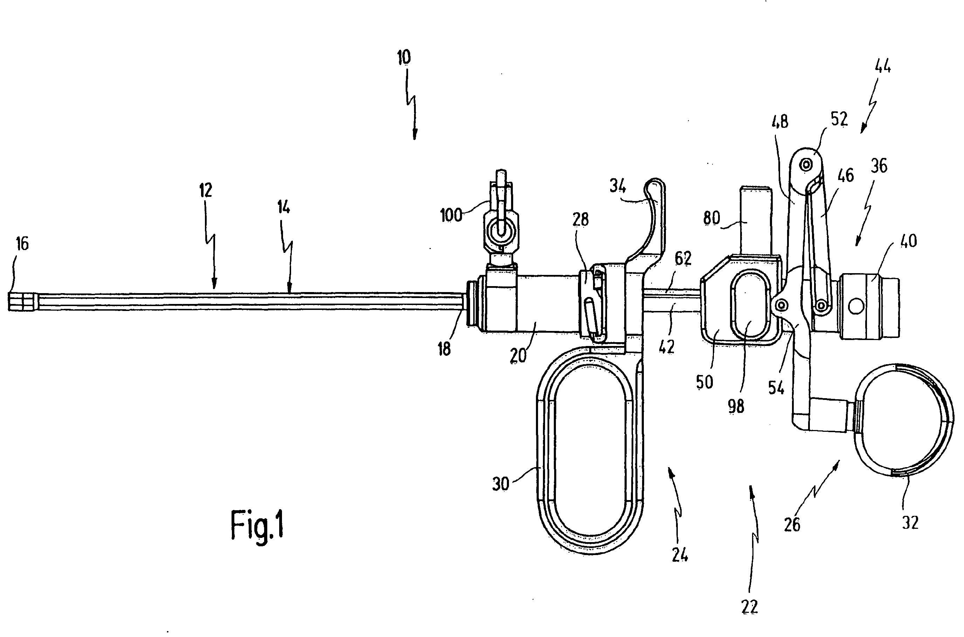

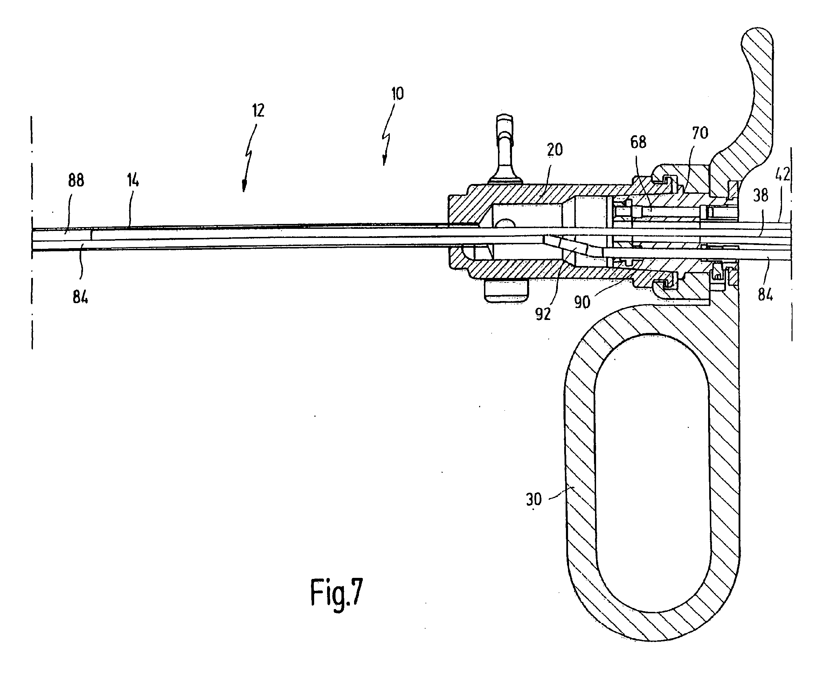

In Figuren 1 bis 8 ist ein mit dem allgemeinen Bezugszeichen 10 versehenes medizinisches Instrument in Form eines Resektoskops dargestellt, das vorzugsweise bei minimal-invasiven chirurgischen Eingriffen auf dem Gebiet der Urologie verwendet wird. Mit dem Instrument 10 kann insbesondere Gewebe der Prostata geschnitten werden.

In Fig. 1 bis 4 ist das Instrument 10 in einer ersten Betriebsart dargestellt, in der es als elektrochirurgisches Instrument einsetzbar ist. In dieser Betriebsart kann mit dem Intstrument 10 das zuvor erwähnte Gewebe unter der Wirkung von Hochfrequenzstrom behandelt werden.

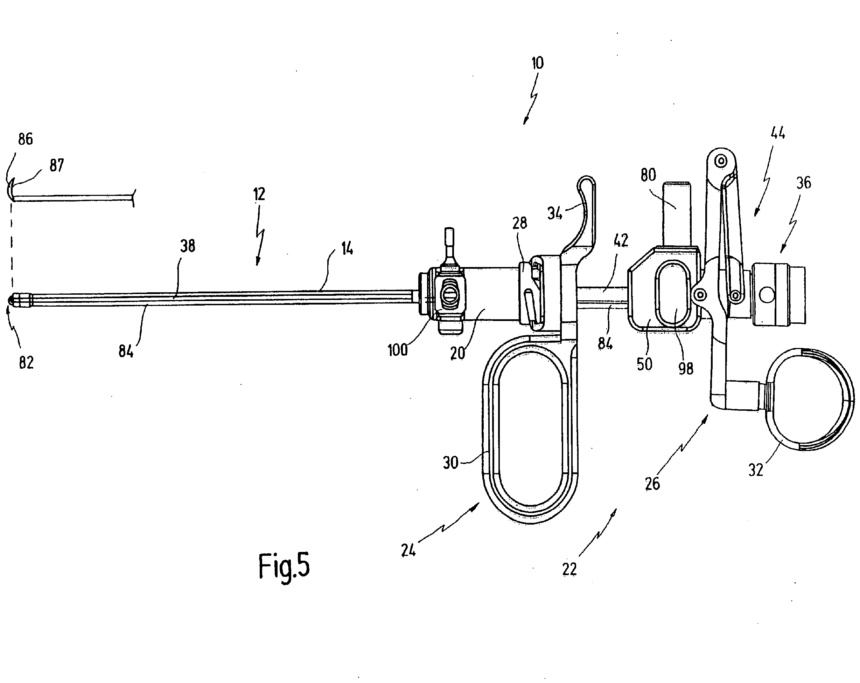

In der Betriebsart in Fig. 5 bis 8 wird das Instrument 10 als stromlos betriebenes Instrument eingesetzt, d.h. als Instrument, mit dem das zuvor erwähnte Gewebe bspw. rein mechanisch geschnitten werden kann.

Zunächst wird mit Bezug auf Fig. 1 bis 4 das Instrument 10 in der zuvor genannten ersten Betriebsart beschrieben.

Das Instrument 10 weist einen langerstreckten Schaft 12 auf, der als an beiden Enden offenes Hohlrohr 14 ausgebildet ist. Das Hohlrohr 14 weist ein distales Ende 16 und ein proximales Ende 18 auf. Das proximale Ende 18 des Hohlrohrs 14 ist mit einer durchmessergrößeren Kupplungshülse 20 fest verbunden.

Das Instrument 10 weist proximal von dem Schaft 12 eine Handhabe 22 auf, die ein unbewegliches Griffteil 24 und ein bewegliches Griffteil 26 aufweist. "Unbeweglich" bedeutet, dass das Griffteil 24 relativ zum Schaft 12 unbeweglich ist, und "beweglich" bedeutet, dass das Griffteil 26 relativ zu dem Schaft 12 beweglich ist. Der Schaft 12 ist über die Kupplungshülse 20 mittels eines Bajonettverschlusses 28 mit der Handhabe 22, genauer gesagt mit dem unbeweglichen Teil 24 verbunden und kann durch Lösen des Bajonettverschlusses 28 entsprechend von der Handhabe 22 abgenommen werden.

Das unbewegliche Griffteil 24, das distal von dem beweglichen Griffteil 26 angeordnet ist, weist einen Ring 30 zum Durchstecken des Mittel- und Ringfingers auf, und das bewegliche Griffteil 26 weist einen Ring 32 zum Durchstecken des Daumens auf. Am unbeweglichen Griffteil 24 ist ferner ein Fortsatz 34 vorhanden, der der Abstützung des Zeigefingers der selben Hand dient, mit der die Handhabe 22 im bestimmungsgemäßen Gebrauch des Instruments 10 gegriffen wird.

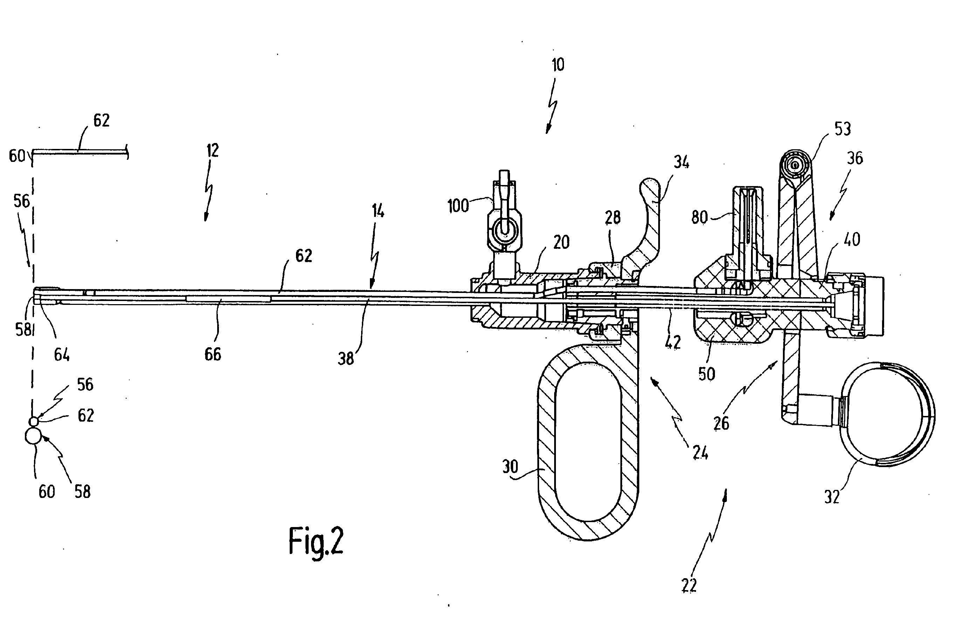

Ebenfalls zum Schaft 12 relativ unbeweglich ist eine Aufnahme 36 für ein Endoskop, von dem in Fig. 2 bis 4 lediglich ein Endoskopschaft 38 dargestellt ist, der im am Instrument 10 montierten Zustand des Endoskops sich vom proximalen Ende des Instruments 10 bis etwa zum distalen Ende 16 oder in Nähe des distalen Endes 16 des Hohlrohrs 14 erstreckt. Ein am proximalen Ende eines solchen Endoskops vorhandenes Okular oder ein Fassungsanschluss zum Anschließen einer Videokamera sind in der Zeichnung nicht dargestellt.

Die Aufnahme 36 für das Endoskop weist am proximalen Ende eine Kupplung 40 auf, um das Endoskop am Instrument 10 festzulegen. Die Aufnahme 36 weist ausgehend von der Kupplung 40 ein Aufnahmerohr 42 auf, das gemäß Fig. 2 mit dem unbeweglichen Griffteil 24 fest verbunden ist, so dass auch die Kupplung 40 der Aufnahme 36 mit dem unbeweglichen Griffteil 24 fest verbunden und somit relativ zum Schaft 12 unbeweglich ist.

Das bewegliche Griffteil 26 ist über eine Gelenkhebelanordnung 44, die einen ersten Gelenkhebel 46 und einen zweiten Gelenkhebel 48 aufweist, einerseits mit der Aufnahme 36 für das Endoskop und andererseits mit einem Schlitten 50 verbunden. Dazu ist der Gelenkhebel 46 distalseitig von der Kupplung 40 an der Aufnahme 36 angelenkt, mit dem Gelenkhebel 48 über ein Gelenk 52 verbunden, während der Gelenkhebel 48 über ein Gelenk 54 an dem Schlitten 50 angelenkt ist.

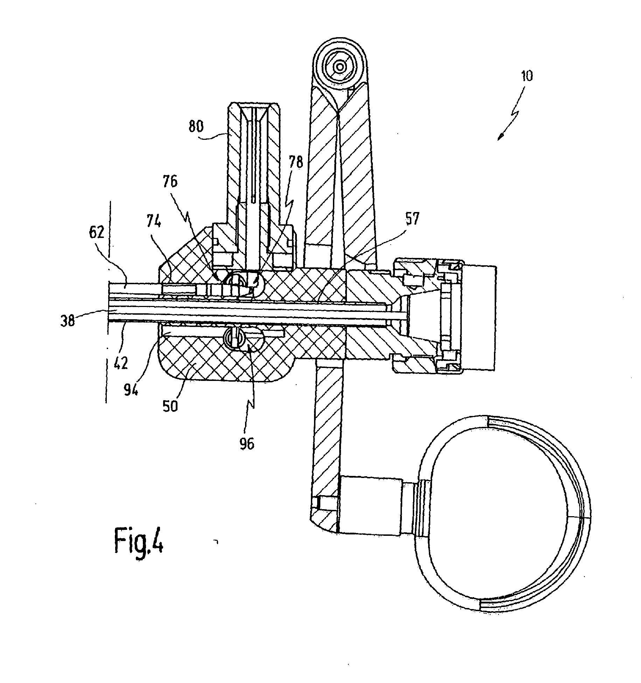

Der Schlitten 50 ist auf dem Aufnahmerohr 42 als Führungsrohr in Längsrichtung des Aufnahmerohrs 42 axial beweglich gelagert. Dazu weist der Schlitten 50 gemäß Fig. 4 eine durchgehende Längsbohrung 57 auf, durch die das Aufnahmerohr 42 hindurchgeht. Der Schlitten 50 ist somit auf dem Aufnahmerohr 42 gleitend verschiebbar.

In Fig. 1, 2 und 4 ist der Schlitten 50 in seiner maximal proximalen Position dargestellt. In dieser Stellung sind die Griffteile 24 und 26 der Handhabe 22 maximal voneinander beabstandet. Durch Bewegen des beweglichen Griffteils 26 in Richtung auf das unbewegliche Griffteil 24 zu, wird der Schlitten 50 auf dem Rohr 42 nach distal verschoben, wobei die distale Endstellung des Schlittens 50 durch die Anlage an dem unbeweglichen Griffteil 24 als Anschlag definiert ist. Das Griffteil 26 ist ferner über eine Torsionsfeder 53 (Fig. 2) im Gelenk 52 in seine proximale Endstellung gemäß Fig. 1, 2 und 4 vorgespannt.

Das Instrument 10 weist in der Betriebsart gemäß Fig. 1 bis 4 weiterhin ein erstes Arbeitselement 56 auf, das als mit Hochfrequenzstrom beaufschlagbare Elektrode 58 ausgebildet ist. In dem vorliegenden Ausführungsbeispiel ist die Elektrode 58 als Resektionsschlinge 60 ausgebildet, die in Fig. 2 zur Verdeutlichung in der oberen Zusatzdarstellung in Seitenansicht und in der unteren Zusatzdarstellung in Vorderansicht gezeigt ist. Das Arbeitselement 56 ist über einen Arbeitselementträger 62, der sich von dem Arbeitselement 56 durch den Schaft 12 hindurch in dessen Längsrichtung und parallel zum Endoskopschaft 38 bis zum Schlitten 50 erstreckt, mit dem Schlitten 50 lösbar verbunden. In der in Fig. 1 und 2 dargestellten maximal proximalen Stellung des Schlittens 50 liegt das Arbeitselement 56 etwa am distalen Ende 16 des Hohlrohrs 14 des Schafts 12 distal nahe vor dem Sichtfenster 64 des Endoskopschafts 38.

Der Arbeitselementträger 62 ist in Form eines dünnen Stabes ausgebildet, der über eine mit dem Arbeitselementträger 62 fest verbundene Führungshülse 66, durch die der Endoskopschaft 38 hindurchgeht, an dem steiferen Endoskopschaft 38 axial geführt ist. Da der Arbeitselementträger 62 mit dem Schlitten 50 verbunden ist, wie hiernach noch näher beschrieben wird, führt eine Betätigung des beweglichen Griffteils 26 über eine axiale Verschiebung des Schlittens 50 zu einer Verschiebung des Arbeitselementträgers 62 und damit des Arbeitselements 56 in Längsrichtung des Schafts 12 relativ zu dem Endoskopschaft 38 und dem Schaft 12.

Der Arbeitselementträger 62 ist, da das Arbeitselement 56 als Elektrode 58 ausgebildet ist, stromleitend ausgebildet, wobei der Arbeitselementträger 62 nach außen entsprechend isoliert ist.

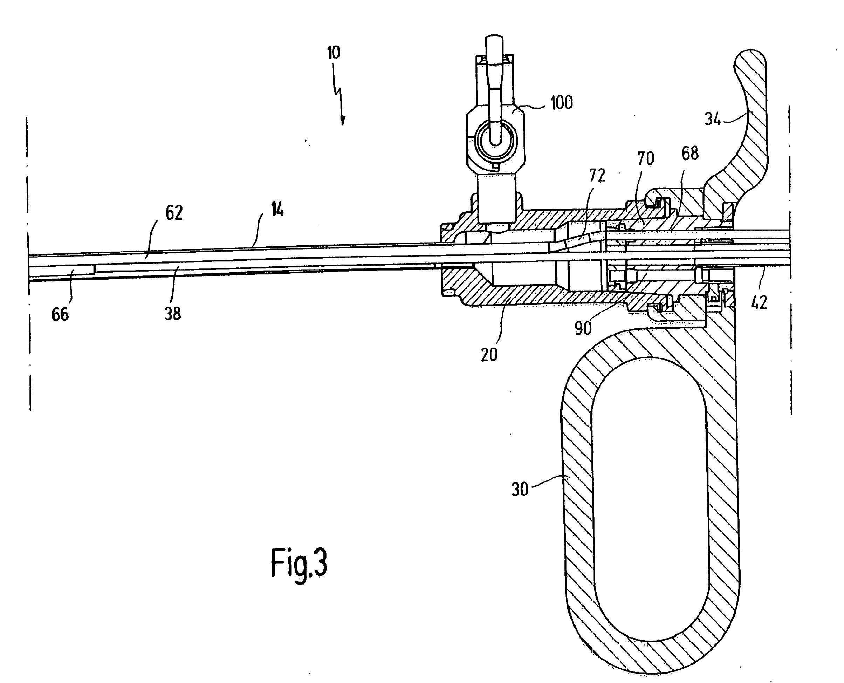

Der Arbeitselementträger 62 ist gemäß Fig. 3 durch die Kupplungshülse 20 und eine erste Bohrung 68 einer mit dem unbeweglichen Griffteil 24 fest verbundenen Buchse 70 durchgeführt. Vor dem Eintritt in die Bohrung 68 weist der Arbeitselementträger 62 eine Kröpfung 72 auf. Gemäß Fig. 4 verläuft der Arbeitselementträger 62 von der Buchse 70 parallel zum Aufnahmerohr 42 für das Endoskop zu dem Schlitten 50, der eine erste Aufnahme 74 aufweist, die in Form einer im Wesentlichen sacklochförmigen Bohrung ausgebildet ist. In der ersten Aufnahme 74 ist das proximale Ende des Arbeitselementträgers 72 festgelegt, und zwar mittels eines selbsttätig schließenden Rastmechanismus 76. Durch Lösen bzw. Entsperren des Rastmechanismus 76 kann das Arbeitselement 56, das fest mit dem Arbeitselementträger 62 verbunden ist, von dem Schlitten 50 getrennt und entnommen werden. Zum Festlegen des Arbeitselementträgers 62 an dem Schlitten 50 wird das proximale Ende des Arbeitselementträgers 62 einfach in die Aufnahme 74 eingesteckt, bis der Rastmechanismus 76 selbsttätig verrastet. In nicht näher dargestellter Weise weist der Rastmechanismus 76 dazu einen federbelasteten Stempel auf, der über eine schräge Fläche am proximalen Ende des Arbeitselementträgers 62 weggedrückt wird, bis der Stempel in eine Einschnürung am proximalen Ende des Arbeitselementträgers 62 einrastet.

Die erste Aufnahme 74 weist weiterhin eine elektrische Kontaktierung 78 auf, die einen oder mehrere elektrische Kontakte aufweist, die dazu dient, den Arbeitselementträger 62 im Bereich seines proximalen Endes elektrisch zu kontaktieren. Der Schlitten 50 weist dazu weiterhin einen Anschluss 80 zum Anschließen eines Stromkabels (nicht dargestellt) auf, über das das Instrument 10 mit einer Hochfrequenzstrom- bzw. Spannungsquelle verbunden werden kann. Der Anschluss 80 bewegt sich bei der Bewegung des Schlittens 50 mit.

Mit der Resektionsschlinge 60 kann durch Beaufschlagen der Schlinge 60 mit Hochfrequenzstrom durch Betätigen des beweglichen Griffteils 26 ein ziehender Schnitt unter der Wirkung von Hochfrequenzstrom durchgeführt werden.

Die erste Aufnahme 74 befindet sich an dem Schlitten 50 oberhalb des Aufnahmerohrs 42 für das Endoskop, wenn das Instrument 10 bestimmungsgemäß zur Durchführung eines Behandlungsvorganges in der Hand gehalten wird. Diese Stellung entspricht der Darstellung des Instruments 10 in Fig. 1 bis 4. Anders ausgedrückt ist die erste Aufnahme 74 in Bezug auf den Endoskopschaft 38 bzw. dessen Längsmittelachse auf der den Griffteilen 24 und 26 abgewandten Seite des Endoskopschafts 38 angeordnet.

Der Arbeitselementträger 62 verläuft über seine gesamte Länge oberhalb des Endoskopschafts 38, und das Arbeitselement 56 in Form der Elektrode 58 steht von dem Arbeitselementträger 62 etwa senkrecht zum Arbeitselementträger 62 nach unten ab, so dass im Blickfeld des Endoskops durch das Sichtfenster 64 im Wesentlichen nur die Elektrode 58 in Form der Resektionsschlinge 60 zu sehen ist.

In Fig. 5 bis 8 ist das Instrument 10 in einer zweiten Betriebsart dargestellt, in der das Arbeitselement 56 samt dem Arbeitselementträger 62 von dem Instrument 10 abgenommen ist, während nun ein zweites Arbeitselement 82 am Instrument 10 angebracht ist, das über einen zweiten Arbeitselementträger 84 an dem Schlitten 50 festgelegt ist.

Das Arbeitselement 82 ist in Form eines Messers 86 ausgebildet, das etwa quer von dem Arbeitselementträger 84 absteht und etwa die Form einer Sichel mit einer konkav gekrümmten Schneide 87 aufweist.

Bis auf den Austausch des Arbeitselements 56 nebst Arbeitselementträger 62 gegen das Arbeitselement 82 mit Arbeitselementträger 84 ist das Instrument 10 gemäß Fig. 5 bis 8 identisch mit dem Instrument 10 in Fig. 1 bis 4.

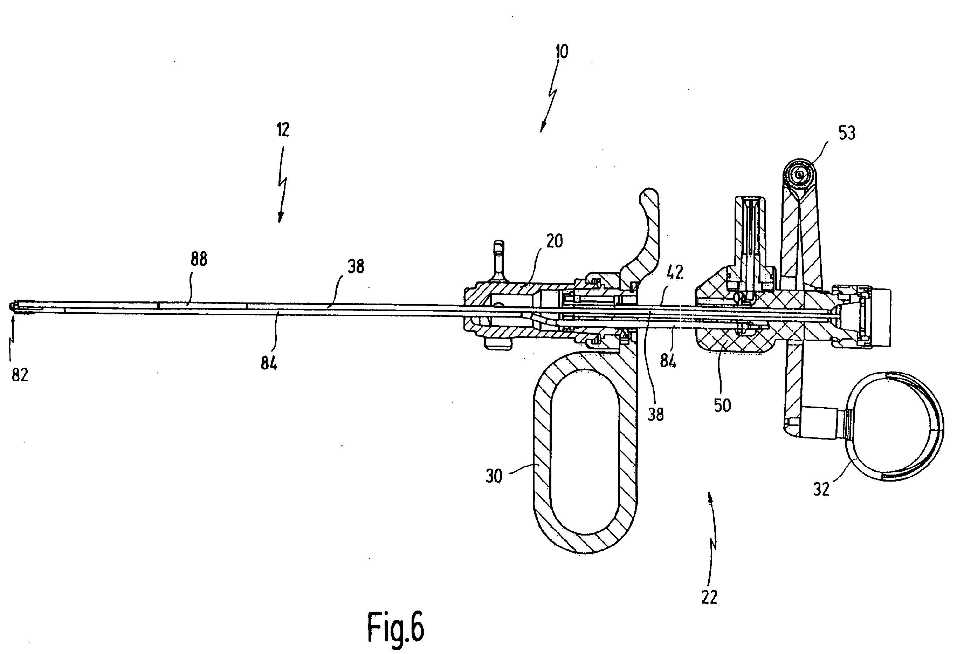

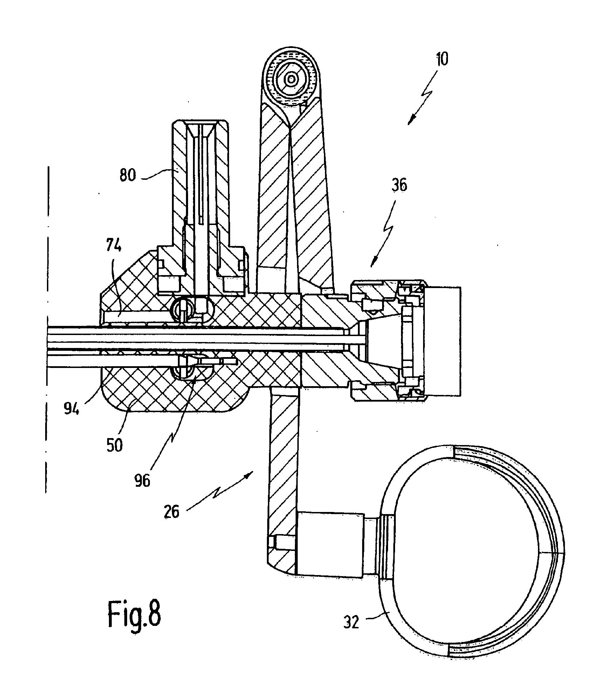

Der Arbeitselementträger 84 ist als langerstreckter dünner Stab ausgebildet, der im Unterschied zu dem Arbeitselementträger 62 nicht als Stromzuführung dient, da das Arbeitselement 82 in Form des Messers 86 stromlos in der Art eines Skalpells arbeitet. Gemäß Fig. 6 ist der Arbeitselementträger 84 parallel zum Endoskopschaft 38 in dem Schaft 12 angeordnet und mittels einer Führungshülse 88, durch die der Endoskopschaft 38 hindurchgeht, an dem Endoskopschaft 38 axial geführt. Der Arbeitselementträger 84 erstreckt sich durch die Kupplungshülse 20 und durch eine zweite Bohrung 90 in der Buchse 70 hindurch. Vor dem Eintritt in die Bohrung 90 weist der Arbeitselementträger 84 eine Kröpfung 92 auf. Von der Buchse 70 erstreckt sich der Arbeitselementträger 84 dann gemäß Fig. 8 bis zu dem Schlitten 50, an dem eine zweite Aufnahme 94 vorhanden ist, in der das proximale Ende des Arbeitselementträgers 84 über einen Rastmechanismus 96 verrastet und damit festgelegt ist.

Die zweite Aufnahme 94 ist gegen die erste Aufnahme 74 elektrisch isoliert und weist selbst keine elektrische Kontaktierung für den Arbeitselementträger 84 auf. Die elektrische Isolierung der zweiten Aufnahme 94 gegen die erste Aufnahme 74 ist dadurch realisiert, dass der Körper des Schlittens 50 insgesamt aus Kunststoff gefertigt ist, wobei in der ersten Aufnahme 74 wie oben beschrieben entsprechend metallische Kontakte zur elektrischen Kontaktierung des ersten Arbeitselementträgers 62 vorhanden sind, die in den Anschluss 80 übergehen.

Während das zweite Arbeitselement 82 mittels seines Arbeitselementträgers 84 auch in der ersten Aufnahme 94 festgelegt werden könnte, hat das Vorsehen der zweiten Aufnahme 94 und die Festlegung des Arbeitselementträgers 84 in der Aufnahme 94, die gegen die erste Aufnahme 74 elektrisch isoliert und selbst keine elektrische Kontaktierung aufweist, den Vorteil, dass vermieden wird, dass Hochfrequenzstrom über den Arbeitselementträger 84 und über das Arbeitselement 82 auf den Patienten bzw. Arzt geleitet wird, wenn nämlich das Instrument 10 am Hochfrequenzgenerator angeschlossen und Letzterer nicht ausgeschaltet ist.

Die zweite Aufnahme 94 ist von der ersten Aufnahme 74 beabstandet, und ist im bestimmungsgemäßen Gebrauch des Instruments 10, also in der in Fig. 5 bis 8 dargestellten Stellung, unterhalb des Aufnahmerohrs 42 für das Endoskop angeordnet. Mit anderen Worten befindet sich die zweite Aufnahme 94 auf der den Griffteilen 24 bis 26, genauer gesagt den Ringen 30 und 32 zugewandten Seite des Endoskopschafts 38. Von dem Schlitten bis zum distalen Ende verläuft der Arbeitselementträger 84 unterhalb des Endoskopschafts 38 bzw. auf der den Ringen 30, 32 zugewandten Seite des Endoskopschafts 32, und das Arbeitselement 82 in Form des Messers 86 steht von dem Arbeitselementträger 84 nach oben ab, so dass durch das Sichtfenster 64 des Endoskops im Wesentlichen nur das Arbeitselement 82 zu sehen ist. Durch Betätigen des beweglichen Griffteils 26 wird das zweite Arbeitselement 82 wie oben für das erste Arbeitselement 56 beschrieben axial bewegt, und mit der konkaven Schneide 87 kann durch Zurückziehen des Arbeitselements 82 ein ziehender Schnitt in das zu behandelnde Gewebe eingebracht werden.

Der Rastmechanismus 96 zum Festlegen des Arbeitselementträgers 84 in der zweiten Aufnahme 94 ist identisch oder ähnlich zu dem Rastmechanismus für die erste Aufnahme 74 ausgestaltet, der bereits oben beschrieben wurde.

Sowohl für den Rastmechanismus 96 als auch für den Rastmechanismus 76 ist ein gemeinsames Bedienelement 98 in Form einer Drucktaste oder eines Druckknopfes vorgesehen, der beim Drücken gleichzeitig beide Rastmechanismen 76 und 96 löst, so dass der jeweils eingesetzte Arbeitselementträger 62 oder 84 vom Schlitten 50 abgezogen werden kann.

Das Instrument 10 wird stets nur mit einem Arbeitselement verwendet, im gezeigten Ausführungsbeispiel also entweder mit dem elektrochirurgischen Arbeitselement 56 mit dem Arbeitselementträger 62 oder dem stromlos arbeitenden Arbeitselement 82 mit dem Arbeitselementträger 84.

Das Instrument 10 weist weiterhin noch einen Saug- und/oder Spülanschluss 100 auf, der mit der Kupplungshülse 20 verbunden ist, und durch den Spülflüssigkeit in das Hohlrohr 14 nach distal und/oder durch das Hohlrohr 14 Flüssigkeit und/oder Gewebestücke abgesaugt werden können.

In Fig. 5 bis 8 ist der Saug- und/oder Spülanschluss 100 um 90° um die Längsmittelachse des Schafts 12 verdreht dargestellt, wobei es sich jedoch versteht, dass der Schaft 12 im praktischen Einsatz des Instruments 10 in der Betriebsart gemäß Fig. 1 bis 4 und in der Betriebsart gemäß Fig. 5 bis 8 die gleiche Drehstellung aufweist. Die unterschiedliche Drehstellung wurde nur in der Zeichnung gewählt.

Während in der Zeichnung nur ein Anschluss 100 gezeigt ist, können auch zwei derartige Anschlüsse vorhanden sein, so dass mit dem Instrument gleichzeitig gesaugt und gespült werden kann.

## Patentansprüche

1. Medizinisches Instrument, insbesondere Resektoskop, mit einem distalen ersten Arbeitselement (56) zur Behandlung von Körpergewebe, das über einen langerstreckten Arbeitselementträger (62) mit einer Handhabe (22) an einem proximalen Ende zum Bewegen des Arbeitselements (56) verbindbar ist, wobei die Handhabe (22) einen in Längsrichtung des Arbeitselementträgers (62) beweglichen Schlitten (50) aufweist, der eine erste Aufnahme (74) zum lösbaren Festlegen des Arbeitselementträgers (62) an dem Schlitten (50) aufweist, und mit einer Aufnahme (36) für ein Endoskop, dessen Endoskopschaft (38) sich im montierten Zustand im wesentlichen parallel zum ersten Arbeitselementträger (62) erstreckt, wobei der Schlitten (50) zumindest eine von der ersten Aufnahme (74) beabstandete zweite Aufnahme (94) für den ersten Arbeitselementträger (62) oder zumindest einen zweiten, am distalen Ende zumindest ein zweites Arbeitselement (82) tragenden Arbeitselementträger (84) aufweist, wobei die erste Aufnahme (74) des Schlittens (50) mit einer elektrischen Kontaktierung (78) versehen ist und die zweite Aufnahme (94) des Schlittens (50) gegen die erste Aufnahme (74) elektrisch isoliert ist, **dadurch gekennzeichnet, dass** die zweite Aufnahme (94) keine elektrische Kontaktierung aufweist.

2. Instrument nach Anspruch 1, **dadurch gekennzeichnet, dass** die zumindest zweite Aufnahme (94) des Schlittens (50) gegen die erste Aufnahme (74) des Schlittens (50) mittels eines elektrisch isolierenden Kunststoffs isoliert ist.

3. Instrument nach Anspruch 1 oder 2, **dadurch gekennzeichnet, dass** der Schlitten (50) insgesamt als Kunststoffkörper ausgebildet ist, in dem in der ersten Aufnahme (74) ein oder mehrere elektrische Kontakte vorhanden sind.

4. Instrument nach einem der Ansprüche 1 bis 3, **dadurch gekennzeichnet, dass** die erste und die zumindest zweite Aufnahme (74, 94) an dem Schlitten (50) so positioniert sind, dass sich, wenn das Instrument (10) bestimmungsgemäß in der Hand gehalten wird, die erste Aufnahme (74) oberhalb eines Aufnahmerohrs (42) für den Endoskopschaft (38) und die zumindest zweite Aufnahme (94) unterhalb des Aufnahmerohrs (42) für den Endoskopschaft (38) befindet.

5. Instrument nach einem der Ansprüche 1 bis 4, **dadurch gekennzeichnet, dass** der erste Arbeitselementträger (62) im am Schlitten (50) festgelegten Zustand, und wenn das Instrument (10) bestimmungsgemäß in der Hand gehalten wird, oberhalb des Endoskopschafts (38) verläuft, und dass das erste Arbeitselement (56) etwa senkrecht zur Längsrichtung des ersten Arbeitselementträgers (62) vom Arbeitselementträger (62) nach unten absteht.

6. Instrument nach einem der Ansprüche 1 bis 5, **dadurch gekennzeichnet, dass** der zumindest zweite Arbeitselementträger (84) im am Schlitten (50) festgelegten Zustand, und wenn das Instrument (10) bestimmungsgemäß in der Hand gehalten wird, unterhalb des Endoskopschafts (38) verläuft, und dass das zumindest eine zweite Arbeitselement (82) etwa senkrecht zur Längsrichtung des zweiten Arbeitselementträgers (84) vom Arbeitselementträger (84) nach oben abstebt.

7. Instrument nach Anspruch 5 oder 6, **dadurch gekennzeichnet, dass** das erste Arbeitselement (56) eine Elektrode (58) ist, die vorzugsweise in Form einer Schlinge (60) ausgebildet ist, und/oder dass das zumindest zweite Arbeitselement (82) ein Messer (86) ist.

8. Instrument nach einem der Ansprüche 1 bis 7, **dadurch gekennzeichnet, dass** die erste Aufnahme (74) und/oder die zumindest zweite Aufnahme (94) des Schlittens (50) einen Rastmechanismus (76, 96) zum Festlegen des ersten oder zumindest zweiten Arbeitselementträgers (62, 84) aufweist/aufweisen.

9. Instrument nach Anspruch 8, **dadurch gekennzeichnet, dass** die erste Aufnahme (74) und die zumindest zweite Aufnahme (94) jeweils einen Rastmechanismus (76, 96) aufweisen, denen zusammen ein gemeinsames Bedienelement (98) zum Lösen des ersten bzw. zumindest zweiten Arbeitselementträgers (62, 84) zugeordnet ist.

10. Instrument nach einem der Ansprüche 1 bis 9, **dadurch gekennzeichnet, dass** an der Handhabe (22) ein Schaft (12) festlegbar ist, der im festgelegten Zustand den ersten oder zumindest zweiten Arbeitselementträger (62, 84) umgibt, und relativ zu dem der erste oder der zumindest zweite Arbeitselementträger (62, 84) axial beweglich ist.

11. Instrument nach einem der Ansprüche 1 bis 10, **dadurch gekennzeichnet, dass** der Schlitten (50) auf einem Führungsrohr (42) axial beweglich gelagert ist, und dass das Führungsrohr (42) als Aufnahmerohr für den Endoskopschaft (38) dient.

## Claims

1. A medical instrument, in particular resectoscope, comprising a distal first work element (56) for treating body tissue, which can be connected via an elongate work element holder (62) to a handle (22) at a proximal end for moving the work element (56), the handle (22) having a carriage (50) which is movable in the longitudinal direction of the work element holder (62) and which has a first seat (74) for releasable attachment of the work element holder (62) to the carriage (50), and comprising a receiver (36) for an endoscope whose endoscope shaft (38), in the assembled state, extends substantially parallel to the first work element holder (62), the carriage (50) having at least a second seat (94) which is spaced apart from the first seat (74) and provided for the first work element holder (62) or for at least a second work element holder (84) which at the distal end supports at least a second work element (82), the first seat (74) of the carriage (50) being provided with an electrical contact arrangement (78), and the second seat (94) of the carriage (50) being electrically insulated relative to the first seat (74), **characterized in that** the second seat (94) has no electrical contact arrangement.

2. The instrument of claim 1, **characterized in that** the at least second seat (94) of the carriage (50) is insulated relative to the first seat (74) of the carriage (50) by means of an electrically insulating plastic.

3. The instrument of claim 1 or 2, **characterized in that** the carriage (50) as a whole is designed as a plastic body in which one or more electrical contacts are present in the first seat (74).

4. The instrument of anyone of claims 1 through 3, **characterized in that** the first and at least second seat (74, 94) on the carriage (50) are positioned such that, when the instrument (10) is held in the hand in a due manner, the first seat (74) is located above a receiving tube (42) for the endoscope shaft (38), and the at least second seat (94) is located below the receiving tube (42) for the endoscope shaft (38).

5. The instrument of anyone of claims 1 through 4, **characterized in that,** when the instrument (10) is held in the hand in a due manner, the first work element holder (62), in the state when secured on the carriage (50), extends above the endoscope shaft (38), and **in that** the first work element (56) protrudes downward from the work element holder (62), approximately perpendicular to the longitudinal direction of the first work element holder (62).

6. The instrument of anyone of claims 1 through 5, **characterized in that,** when the instrument (10) is held in the hand in a due manner, the at least second work element holder (84), in the state when secured on the carriage (50), extends below the endoscope shaft (38), and **in that** the at least second work element (82) protrudes upward from the work element holder (84), approximately perpendicular to the longitudinal direction of the second work element holder (84).

7. The instrument of claim 5 or 6, **characterized in that** the first work element (56) is an electrode (58) which is preferably configured in the form of a loop (60), and/or **in that** the at least second work element (82) is a blade (86).

8. The instrument of anyone of claims 1 through 7, **characterized in that** the first seat (74) and/or the at least second seat (94) of the carriage (50) has/have a lock mechanism (76, 96) for securing the first or at least second work element holder (62, 84).

9. The instrument of claim 8, **characterized in that** the first seat (74) and the at least second seat (94) each have a lock mechanism (76, 96), and the latter are jointly assigned a common operating element (98) for releasing the first and at least second work element holder (62, 84).

10. The instrument of anyone of claims 1 through 9, **characterized in that** a shaft (12) can be secured on the handle (22) and, in the secured state, this shaft (12) surrounds the first or at least second work element holder (62, 84), and the first or the at least second work element holder (62, 84) is axially movable relative to said shaft (12).

11. The instrument of anyone of claims 1 through 10, **characterized in that** the carriage (50) is mounted in an axially movable manner on a guide tube (42), and **in that** the guide tube (42) serves as a receiving tube for the endoscope shaft (38).

## Revendications

1. Instrument médical, en particulier, résectoscope, comportant un premier élément de travail (56) distal pour le traitement de tissus corporels, qui peut être relié par le biais d'un support d'élément de travail (62) allongé, à une poignée (22) sur une extrémité proximale pour mobiliser l'élément de travail (56), la poignée (22) présentant un chariot (50) mobile dans le sens longitudinal du support d'élément de travail (62), qui présente un premier élément de réception (74) pour la fixation amovible du support d'élément de travail (62) sur le chariot (50) et comportant un élément de réception (36) d'un endoscope, dont la tige d'endoscope (38) s'étend à l'état monté, essentiellement parallèlement au premier support d'élément de travail (62), le chariot (50) présentant au moins un deuxième élément de réception (94) à distance du premier élément de réception (74) pour le premier support d'élément de travail (62) ou au moins un deuxième support d'élément de travail (84) portant sur l'extrémité distale, au moins un deuxième élément de travail (82), le premier élément de réception (74) du chariot (50) étant doté d'un contact électrique (78) et le deuxième élément de réception (94) du chariot (50) étant isolé électriquement du premier élément de réception (74), **caractérisé en ce que** le deuxième élément de réception (94) ne présente pas de contact électrique.

2. Instrument selon la revendication 1, **caractérisé en ce qu'**au moins le deuxième élément de réception (94) du chariot (50) est isolé du premier élément de réception (74) du chariot (50) au moyen d'une matière plastique électriquement isolante.

3. Instrument selon la revendication 1 ou 2, **caractérisé en ce que** le chariot (50) est constitué globalement comme un corps en matière plastique dans lequel, dans le premier élément de réception (74) sont présents un ou plusieurs contacts électriques.

4. Instrument selon l'une des revendications 1 à 3, **caractérisé en ce que** le premier et au moins le deuxième élément de réception (74, 94) sont positionnés sur le chariot (50) de telle sorte que l'instrument (10) est tenu dans la main de manière déterminée, le premier élément de réception (74) se trouvant au-dessus d'un tube de réception (42) de la tige d'endoscope (38) et au moins le deuxième élément de réception (94) se trouvant sous le tube de réception (42) de la tige d'endoscope (38).

5. Instrument selon l'une des revendications 1 à 4, **caractérisé en ce que** le premier support d'élément de travail (62) évolue à l'état fixé sur le chariot (50) et lorsque l'instrument (10) est tenu dans la main de façon déterminée, évolue au-dessus de la tige de l'endoscope (38), et **en ce que** le premier élément de travail (56) dépasse vers le bas à peu près verticalement par rapport à la direction longitudinale du premier support d'élément de travail (62) du support d'élément de travail (62).

6. Instrument selon l'une des revendications 1 à 5, **caractérisé en ce qu'**au moins le deuxième support d'élément de travail (84) à l'état fixé dans le chariot (50) et lorsque l'instrument (10) est tenu dans la main de façon déterminée, évolue sous la tige de l'endoscope (38) et **en ce qu'**au moins un deuxième élément de travail (82) dépasse vers le haut, à peu près perpendiculairement par rapport à la direction longitudinale du deuxième support d'élément de travail (84) du support d'élément de travail (84).

7. Instrument selon la revendication 5 ou 6, **caractérisé en ce que** le premier élément de travail (56) est une électrode (58) qui est constituée de préférence sous la forme d'une anse (60) et/ou **en ce que** au moins le deuxième élément de travail (82) est un couteau (86).

8. Instrument selon l'une des revendications 1 à 7, **caractérisé en ce que** le premier élément de réception (74) et/ou au moins le deuxième élément de réception (94) du chariot (50) présente(nt) un mécanisme d'encliquetage (76, 96) pour fixer le premier ou au moins le deuxième support d'élément de travail (62, 84).

9. Instrument selon la revendication 8, **caractérisé en ce que** le premier élément de réception (74) et au moins le deuxième élément de réception (94) présentent respectivement un mécanisme d'encliquetage (76, 96) auxquels est affecté conjointement un élément de commande commun (98) pour détacher le premier ou au moins le deuxième support d'élément de travail (62, 84).

10. Instrument selon l'une des revendications 1 à 9, **caractérisé en ce que,** sur la poignée (22) se trouve une tige (12) pouvant être fixée, qui entoure à l'état fixé le premier ou au moins le deuxième élément de travail (62, 84) et est mobile par rapport au premier ou au moins au deuxième support d'élément de travail (62, 84).

11. Instrument selon l'une des revendications 1 à 10, **caractérisé en ce que** le chariot (50) est placé de façon mobile axialement sur un tube de guidage (42) et **en ce que** le tube de guidage (42) sert de tube de réception de la tige d'endoscope (38).
